# EUROPEAN PATENT APPLICATION

(11) **EP 4 394 039 A1**
(43) Date of publication of application: **03.07.2024**
(21) Application number: 21959786.1
(22) Date of filing: 06.12.2021
(51) Int. Cl.: C12N 15/64, C12N 15/75, C12N 1/21, C12P 13/06, C12R 1/07, C12R 1/10

(54) **GENETICALLY ENGINEERED STRAIN CAPABLE OF PRODUCING L-ALANINE, CONSTRUCTION METHOD THEREFOR, AND APPLICATION THEREOF**

(30) Priority: 08.10.2021 CN 202111171829
(71) Applicant: Anhui BBCA Biochemical Co., Ltd., Bengbu, Anhui 233700 (CN)
(72) Inventor: XU, Ping, Shanghai 200240 (CN); HAN, Xiao, Shanghai 200240 (CN); TAO, Fei, Shanghai 200240 (CN); MU, Xiaoling, Bengbu, Anhui 233700 (CN); CHEN, Sihong, Bengbu, Anhui 233700 (CN); LI, Weili, Bengbu, Anhui 233700 (CN)
(74) Representative: Elkington and Fife LLP
(86) International application number: PCT/CN2021/135643
(87) International publication number: WO 2023/056699

(57) **Abstract**

The present invention discloses an L-alanine-producing genetically engineered strain, as well as a method of construction and use thereof, and pertains to the field of bioengineering. According to the present invention, through enhancing the glycolysis pathway or/and introducing a gene for thermostable alanine dehydrogenase, a genetically engineered strain capable of high-yield production of alanine under a high temperature condition of 42 °C to 55 °C can be constructed. Moreover, through knocking out alanine racemase genes, optical purity of L-alanine can be significantly increased. When the original strain possesses a lactate synthesis pathway, blocking this lactate synthesis pathway can augment the proportion of a pyruvate synthesis pathway, resulting in an additionally increased yield of L-alanine. The present invention overcomes the problems of fermentation at a low temperature, high cost and the like, which arise from the use of conventional L-alanine production techniques, enables production of L-alanine by fermentation at a high temperature of 42 °C to 55 °C with a yield of 95 g/L or higher, and is of high value to industrial application.

## Description

### Field of the Invention

The present invention relates to the field of bioengineering and, in particular, to an L-alanine-producing genetically engineered strain, as well as to a method of construction and use thereof.

### Description of the Prior Art

As one of the 20 basic amino acids and one of the smallest chiral molecules, L-alanine is widely used in food, medicine, commodity chemicals and other fields. In the food industry, L-alanine can be added to food as a preservative, sweetener or the like. In the field of medicine and health care, L-alanine can not only be used in amino acid-based nutritional supplements, but also plays an important role in the treatment of acute and chronic renal failure, liver disease, diabetes, obesity and other diseases. In the field of commodity chemical products, L-alanine can be used to synthesize amino acid-based surfactants and provide high bioaffinity.

Production methods of L-alanine mainly include chemical synthesis, enzymatic catalysis and fermentation. A currently predominant chemical synthesis approach is the propionic acid chlorination process using liquid chlorine and propionic acid as the main raw materials. Due to a long synthetic route, high cost and low yield, the chemical synthesis approach has been substantially faded out. Enzymatic catalysis of L-alanine essentially involves decarboxylation of aspartate catalyzed immobilized L-aspartate-β-decarboxylase. However, the enzymatic catalysis approach suffers from low carbon utilization of the four-carbon substrate to the three-carbon product and a relatively high price of aspartate and is therefore not suitable for large-scale production. Principally with glucose as a substrate, L-alanine can be produced by fermentation with a strain constructed by genetic engineering. This approach has attracted widespread attention thanks to the advantages of the inexpensive and easily available raw material, high product yield, low cost and so forth.

Compared with fermentation at a common temperature of 42 °C or lower, high-temperature fermentation can provide a significantly reduced risk of contamination, improved raw material conversion efficiency and reduced heat exchange cost and is therefore of high value to large-scale production by fermentation. In production of alanine by fermentation, genetically engineered *Escherichia coli* strains are often used as producing strains. However, as the optimum growth temperature for these strains is 37 °C, they are not suitable for use in high-temperature fermentation.

Therefore, those skilled in the art are directing their effort toward developing a strain suitable for large-scale production of L-alanine by fermentation under a high-temperature condition.

### Summary of the Invention

In view of the above, the problem sought to be solved by the present invention is to provide a genetically engineered strain capable of producing L-alanine under a high-temperature condition of 42 °C or higher, as well as a method of construction and use thereof.

To achieve the above goal, in a first aspect of the present invention, there is provided a method of constructing an L-alanine-producing genetically engineered strain, which includes the steps of:
providing an original strain possessing a pyruvate synthesis pathway;
engineering the genome of the original strain through any one step, any two steps or three steps of S200, S300 and S400;
S200: inserting a copy of a 6-Phosphofructokinase gene *pfk* and a copy of a pyruvate kinase *gene pyk*;
S300: inserting a gene *GSald* for alanine dehydrogenase thermostable at 42 °C to 55 °C;
S400: inactivating or deleting an alanine racemase gene contained in the genome of the original strain.

An L-alanine-producing genetically engineered strain constructed in accordance with the first aspect of the present invention is capable of L-alanine production by fermentation under a high-temperature condition of 42 °C to 55 °C.

In some embodiments of the present invention, the original strain further possesses a lactate synthesis pathway and the genome of the original strain contains a lactate dehydrogenase gene. In these cases, the method further includes the step of:
S100: inactivating or deleting the lactate dehydrogenase gene in the genome of the original strain.

In a second aspect of the present invention, there is provided a genetically engineered strain constructed in accordance with the method of the first aspect of the present invention.

In a third aspect of the present invention, there is provided use of the genetically engineered strain according to the second aspect of the present invention for L-alanine production.

In a fourth aspect of the present invention, there is provided a method of producing L-alanine, in which the genetically engineered strain according to the second aspect of the present invention is subjected to fermentation culture, wherein the fermentation culture is at a temperature of 42 °C to 55 °C.

In some embodiments of the present invention, the fermentation culture step includes inoculation of activated seeds into fermentation culture medium containing a carbon source and fed-batch fermentation at a fermentation culture temperature of 42 °C to 55 °C and an agitation speed of 50 rpm to 350 rpm to a predetermined broth density.

Benefits offered by the techniques disclosed in the present invention include:
1. The genetically engineered strain of the present invention is capable of L-alanine production at a high temperature (e.g., 42 °C to 55 °C) by a fermentation process, which has the advantages including low energy consumption and resistance to contamination, effectively reducing the production cost.
2. With a thermophilic strain as an original strain, a genetically engineered strain can be constructed, which is capable of producing L-alanine by fermentation at a high temperature of 42 °C to 55 °C (in particular, for example, 50 °C). Moreover, through enhancing the glycolysis pathway or/and introducing thermostable alanine dehydrogenase, a significantly increased yield of alanine can be obtained. Through knocking out alanine racemase genes, production of optically pure L-alanine is made possible. These strageties can be synergistically combined to enable high-yield production of optically pure L-alanine by fermentation at a temperature as high as 42 °C to 55 °C, and the resulting L-alanine typically exhibits an optical purity of 98% or higher, or even 99% or higher. In some examples, the yield of the optically pure L-alanine can be as high as 95 g/L or higher, or even 100 g/L or higher. The present invention overcomes the problems of fermentation at a low temperature, high cost and the like, which arise from the use of conventional L-alanine production techniques, enables production of L-alanine by fermentation at a high temperature, and is of high value to industrial application.
3. In case of the original strain possessing a lactate synthesis pathway, blocking the lactate synthesis pathway can augment the proportion of the pyruvate synthesis pathway, resulting in an increase in the yield of L-alanine. In case of the original strain producing both lactate and pyruvate, blocking the lactate synthesis pathway in the original strain can augment the proportion of the alanine synthesis pathway. Further, through enhancing the glycolysis pathway and introducing thermostable alanine dehydrogenase, a genetically engineered strain capable of high-yield production of alanine can be constructed. Knocking out alanine racemase genes enables production of optically pure L-alanine.
4. For example, *Bacillus licheniformis* usable in fermentation at a high temperature of 50 °C can be used for this purpose. With a *Bacillus licheniformis* BN11 strain capable of high-yield production of D-lactate as a starting strain, a D-lactate synthetic pathway therein may be eliminated to enhance the flux of the glycolysis pathway. Moreover, thermostable heterologous alanine dehydrogenase may be introduced, and a D-alanine synthesis pathway may be eliminated. In this way, high-yield production of optically pure L-alanine can be achieved at a high temperature of 42 °C to 55 °C. The resulting L-alanine typically exhibits an optical purity of 98% or higher, or even 99% or higher. In some examples, the yield of the optically pure L-alanine can be as high as 95 g/L or higher, or even 100 g/L or higher.

Below, the concept, structural details and resulting technical effects of the present application will be further described with reference to the accompanying drawings to provide a full understanding of the objects, features and effects of the invention.

### Brief Description of the Drawing

Other features, objects and advantages of the present invention will become more apparent upon reading the following detailed description of non-limiting examples when taken in conjunction with the accompanying drawings, in which:
Fig. 1 schematically illustrates alanine synthesis and metabolism pathways in an exemplary genetically engineered *Bacillus licheniformis* strain according to an embodiment of the present invention;
Fig. 2 is a plot diagram of L-alanine production by fed-batch fermentation at 50 °C in a 5-L fermenter by an L-alanine-producing genetically engineered strain BLA-1 according to an embodiment of the present invention (▲: OD₆₀₀; ■: glucose; ●: alanine); and
Fig. 3 shows the structure of a pKVM vector used in some embodiments of the present invention, which contains two antibiotic resistance genes: *ampR* (ampicillin resistance) and *ermC* (erythromycin resistance), as well as the *bgaB* gene that encodes galactosidase. All of these genes can be used as selectable markers. This plasmid has a heat-sensitive origin of replication (oriT pE194ts) and origins of replication in *Escherichia coli* (ori) and *Bacillus licheniformis* (oriT).

### Detailed Description of the Preferred Embodiments

Specific embodiments of the present invention will be described in detail below with reference to the accompanying drawings.

In the following, the figures annexed to this specification are referenced to describe a few preferred embodiments and examples of the present invention so that the techniques thereof become more apparent and readily understood. The invention may be embodied and exemplified in many different forms, and its scope is not limited to the embodiments and examples disclosed herein.

It will be understood that the features described hereinabove and the features detailed in the following description (including, but not limited to, the embodiments and examples) can be combined in any suitable manner to create new embodiments, as long as the invention can be implemented.

As used herein, the terms "preferred", "particular", "specific", "further", "furthermore" and the like are used merely to describe embodiments or examples with good effects or with certain degrees of particularity. It will be understood that they are not intended to limit the scope of the invention in any way.

As used herein, "and/or" and "or/and" both means that the listed items are alternatives, but the alternatives also include any combination of the listed items.

As used herein, "a combination thereof, "any combination", "an arbitrary combination" and "in any combination" all mean that the listed items can be combined in any suitable manner as long as the present invention can be implemented. The number of items that can be combined is two or more.

As used herein, any reference to a concentration in percentage should be understood to refer to a final concentration in a system, unless otherwise defined or specified.

As used herein, any numerical range recited is intended to include both the lower and upper limits, unless otherwise specified.

As used herein, "or higher" or "or lower" following a number is intended to refer to a range including the specific number, unless otherwise defined.

The features of the present invention described above and below can be combined in any suitable manner, as long as there is no conflict and the resultant embodiment can be implemented to solve the problem that the present invention seeks to solve. The features can be combined in any suitable manner, as long as the resultant embodiment can solve the problem that the present invention seeks to solve and achieve an expected effect.

As used herein, "starting strain" and "original strain" have the same meaning and can be used interchangeably.

As used herein, any reference to temperature control should be understood to mean that a temperature is controlled so as to remain at a constant value or vary within a range.

As used herein, "high-temperature", when used to describe the production of L-alanine, is relative to a common temperature (lower than 42 °C, typically 37 °C or lower). As used herein, unless otherwise defined, "high-temperature production" is intended to refer to production by fermentation at 42 °C to 55 °C, for example, but not limited to, 42 °C, 43 °C, 44 °C, 45 °C, 46 °C, 47 °C, 48 °C, 49 °C, 50 °C, 52 °C, 53 °C, 54 °C or 55 °C.

As used herein, unless otherwise specified, "pyruvate synthesis pathway" refers to a pathway that synthesizes pyruvate by glycolysis of a carbon source. As used herein, "possessing a pyruvate synthesis pathway" means "having the ability to synthesize pyruvate endogenously".

As used herein, unless otherwise specified, "lactate synthesis pathway" refers to a synthetic pathway that converts pyruvate to lactate. As used herein, unless otherwise specified, "possessing a lactate synthesis pathway" means "having the ability to producing lactate through a lactate synthesis pathway". That is, "possessing a lactate synthesis pathway" means "having the ability to synthesize lactate endogenously".

As used herein, unless otherwise specified, "alanine synthesis pathway" refers to a synthetic pathway that converts pyruvate to alanine. As used herein, unless otherwise specified, "possessing an alanine synthesis pathway" means "having the ability to producing alanine through an alanine synthesis pathway", i.e., "having the ability to synthesize alanine endogenously". As used herein, when something is described as possessing an alanine synthesis pathway, unless otherwise specified, it is intended to mean that it possesses both a pathway that synthesizes L-alanine from pyruvate and a pathway that synthesizes D-alanine from L-alanine.

As used herein, unless otherwise specified, "D-alanine synthesis pathway" refers to a pathway that synthesizes D-alanine from L-alanine, and "possessing a D-alanine synthetic pathway" means "having the ability to synthesize D-alanine endogenously".

As used herein, a gene cluster fragment refers to a fragment containing at least two relevant genes, which may be either identical or different. When describing a gene cluster fragment composed of identical genes, the term "repeated" shall be broadly interpreted as meaning that the genes may be directly adjacent to one another, or a spacer sequence may be present between any two of the genes.

According to the present invention, one of the typically approaches for deleting a relevant gene is to knock it out.

As used herein, unless otherwise specified, "inactivation" and "deletion" may refer to partial or complete inactivation and partial or complete deletion, respectively.

**Table 1. Abbreviations and Full Names in English and Chinese of Some Terms**

| Abbreviation | English | Chinese |
|---|---|---|
| NADH | Nicotinamide Adenine Dinucleotide Hydrogen | 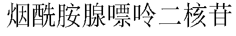 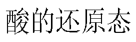 |

**Table 2. Names of Some Terms in English and Chinese**

| English | Chinese |
|---|---|
| *Bacillus licheniformis* | 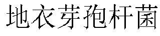 |
| *Bacillus coagulans* | 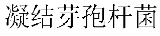 |
| Glucose | 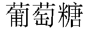 |
| Alanine | 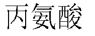 |

The amino acid and nucleotide sequences mentioned herein are summarized in Table 3.

**Table 3. Some Amino Acid and Nucleotide Sequences Mentioned Herein**

| Sequence No. | Type of Sequence | Species of Organism | Characteristic Information |
|---|---|---|---|
| SEQ ID NO. 1 | DNA | Artificial Sequence | Alanine Dehydrogenase Gene *GSald* |
| SEQ ID NO. 2 | DNA | Artificial Sequence | Pₐₗₛ Promoter |
| SEQ ID NO. 3 | DNA | Artificial Sequence | pKVM-XPDdldh-UpF Primer |
| SEQ ID NO. 4 | DNA | Artificial Sequence | XPDdldh-UpR Primer |
| SEQ ID NO. 5 | DNA | Artificial Sequence | XPDdldh-DownF Primer |
| SEQ ID NO. 6 | DNA | Artificial Sequence | pKVM-XPDdldh-DownR Primer |
| SEQ ID NO. 7 | DNA | Artificial Sequence | pKVM-XPDldh-UpF Primer |
| SEQ ID NO. 8 | DNA | Artificial Sequence | Pₐₗₛ-XPDldh-UpR Primer |
| SEQ ID NO. 9 | DNA | Artificial Sequence | XPDldh-Pₐₗₛ-F Primer |
| SEQ ID NO. 10 | DNA | Artificial Sequence | PFYAK-Pₐₗₛ-R Primer |
| SEQ ID NO. 11 | DNA | Artificial Sequence | Pₐₗₛ-PFYAK-F Primer |
| SEQ ID NO. 12 | DNA | Artificial Sequence | XPDldh-PFYAK-R Primer |
| SEQ ID NO. 13 | DNA | Artificial Sequence | PFYAK-XPDldh-DownF Primer |
| SEQ ID NO. 14 | DNA | Artificial Sequence | pKVM-XPDldh-DownR Primer |
| SEQ ID NO. 15 | DNA | Artificial Sequence | Pₐₗₛ-XPDdldh-UpR Primer |
| SEQ ID NO. 16 | DNA | Artificial Sequence | XPDdldh-Pₐₗₛ-F Primer |
| SEQ ID NO. 17 | DNA | Artificial Sequence | XPDdldh-GSald-R Primer |
| SEQ ID NO. 18 | DNA | Artificial Sequence | GSald-XPDdldh-DownF Primer |
| SEQ ID NO. 19 | DNA | Artificial Sequence | pKVM-XPDalrl-UpF Primer |
| SEQ ID NO. 20 | DNA | Artificial Sequence | XPDalr1-UpR Primer |
| SEQ ID NO. 21 | DNA | Artificial Sequence | XPDalrl-DownF Primer |
| SEQ ID NO. 22 | DNA | Artificial Sequence | pKVM-XPDalr1-DownR Primer |
| SEQ ID NO. 23 | DNA | Artificial Sequence | pKVM-XPDalr2-UpF Primer |
| SEQ ID NO. 24 | DNA | Artificial Sequence | XPDalr2-UpR Primer |
| SEQ ID NO. 25 | DNA | Artificial Sequence | XPDalr2-DownF Primer |
| SEQ ID NO. 26 | DNA | Artificial Sequence | pKVM-XPDalr2-DownR Primer |
| SEQ ID NO. 27 | DNA | *Bacillus coagulans* | 6-Phosphofructokinase Gene *pfk* |
| SEQ ID NO. 28 | DNA | *Bacillus coagulans* | Pyruvate Kinase Gene *pyk* |
| SEQ ID NO. 29 | DNA | *Bacillus licheniformis* | Alanine Racemase Gene *alr1* |
| SEQ ID NO. 30 | DNA | *Bacillus licheniformis* | Alanine Racemase Gene *alr2* |
| SEQ ID NO. 31 | DNA | *Bacillus licheniformis* | D-Lactate Dehydrogenase Gene *ldh_{Ti}* |
| SEQ ID NO. 32 | PRT | Artificial Sequence | Alanine Dehydrogenase GSald |

SEQ ID NO. 1 (*GSald*): 
ATGAAAATCGGCATCCCGAAAGAAATCAAAAACAACGAAAACCGCGTCGC
GATCACGCCGGCGGGCGTCATGACGCTGGTCAAAGCGGGCCATGACGTCTA
TGTCGAAACGGAAGCGGGCGCGGGCAGCGGCTTTAGCGACAGCGAATATG
AAAAAGCGGGCGCGGTCATCGTCCCGAACGCGGAAGACGCGTGGACGGC
GGAAATGGTCCTGAAAGTCAAAGAACCGCTGGCGGAAGAATTTCGCTATTT
TCGCCCGGGCCTGATCCTGTTTACGTATCTGCATCTGGCGGCGGCGGAAGC
GCTGACGAAAGCGCTGGTCGAACAGAAAGTCGTCGGCATCGCGTATGAAA
CGGTCCAGCTGGCGAACGGCAGCCTGCCGCTGCTGACGCCGATGAGCGAA
GTCGCGGGCCGCATGAGCGTCCAGGTCGGCGCGCAGTTTCTGGAAAAACC
GCATGGCGGCAAAGGCATCCTGCTGGGCGGCGTCCCGGGCGTCCGCCGCG
GCAAAGTCACGATCATCGGCGGCGGCACGGCGGGCACGAACGCGGCGAA
AATCGCGGTCGGCCTGGGCAGCGACGTCACGATCCTGGACATCAACGCGG
AACGCCTGCGCGAACTGGACGACCTGTTTGGCGACCATGTCACGACGCTG
ATGAGCAACAGCTATCATATCGCGGAATGCGTCCGCGAAAGCGACCTGGTC
GTCGGCGCGGTCCTGATCCCGGGCGCGAAAGCGCCGAAACTGGTCACGGA
AGAAATGGTCCGCAGCATGACGCCGGGCAGCGTCCTGGTCGACATCGCGA
TCGACCAGGGCGGCATCTTTGAAACGACGGACCGCGTCACGACGCATGAC
GACCCGACGTATGTCAAACATGGCGTCGTCCATTATGCGGTCGCGAACATG
CCGGGCGCGGTCCCGCGCACGAGCACGTTTGCGCTGACGAACGTCACGAT
CCCGTATGCGCTGCAGATCGCGAACAAAGGCTATCGCGCGGCGTGCCTGGA
CAACCCGGCGCTGCTGAAAGGCATCAACACGCTGGACGGCCATATCGTCTA
TGAAGCGGTCGCGGCGGCGCATAACATGCCGTATACGGACGTCCATAGCCT
GCTGCATGGCTAA
SEQ ID NO. 2 (Pₐₗₛ promoter): 
AAGGTGACGCCTATTTCACTTTCTAGCTGTTTAATCTGCTGGCTGAGCGGAG
GCTGAGTCATGTTCAGCCGAAGAGCTGCTTTTCCGAAATGCAGTTCTTCGG
CAACAACCATAAAATAACGAAGATGGCGCAGCTCCATTAATCACTCATTCCT
TTCTGAATGCGATTTCAGTCGTTTTACATATTAATTGTAAGACAAAGAAGTA
TTGGAAAACAATTTCCACAAGATGTATATTTAATAATACAATAATTTTATTAA
 AAATTCATTGTAAATGAATGAAAATGGAGGAGTGAGGGCT
SEQ ID NO. 3: CCTCGCGTCGGGCGATATCGGATCCGAAGGGGAAAGTCTTCGATTTCT
SEQ ID NO. 4: AGAGGGCTTTTTCATGCTGAAGAGGTCAAAAAGAGCC
SEQ ID NO. 5: TTTGACCTCTTCAGCATGAAAAAGCCCTCTTTGAAAAG
SEQ ID NO. 6: CCATGGTACCCGGGAGCTCGAATTCCATAAGACCGCTGATGACAAGC
SEQ ID NO. 7: TCCAGCCTCGCGTCGGGCGATATCGTCCCCATAACAACGGAATCATC
SEQ ID NO. 8: AATAGGCGTCACCTTGACTCATCATTCCTTTGCCGTT
SEQ ID NO. 9: AAGGAATGATGAGTCAAGGTGACGCCTATTTCACTTTC
SEQ ID NO. 10: TCCAATTCGCTTCATAGCCCTCACTCCTCCATTTTC
SEQ ID NO. 11: GGAGGAGTGAGGGCTATGAAGCGAATTGGAGTATTGACA
SEQ ID NO. 12: CTTCATGGTGTTCAGTTACAATACAGTCGCATGGCC
SEQ ID NO. 13: GCGACTGTATTGTAACTGAACACCATGAAGATACTAACATCA
SEQ ID NO. 14: ACTAGACAGATCTATCGATGCATGCTTTCCCTTATTCCTTTAAACCCG
SEQ ID NO. 15: AATAGGCGTCACCTTGCTGAAGAGGTCAAAAAGAGCC
SEQ ID NO. 16: TTTGACCTCTTCAGCAAGGTGACGCCTATTTCACTTTCT
SEQ ID NO. 17: AGAGGGCTTTTTCATTTAGCCATGCAGCAGGCTATG
SEQ ID NO. 18: CTGCTGCATGGCTAAATGAAAAAGCCCTCTTTGAAAAG
SEQ ID NO. 19: CCTCGCGTCGGGCGATATCGGATCCAAAATATGACGCTGTCTCAAATTGA
SEQ ID NO. 20: CTAATTCATCAATTTGACACTTCCTGTTCCTTGTTTCACT
SEQ ID NO. 21: GGAACAGGAAGTGTCAAATTGATGAATTAGCGGAAAAAC
SEQ ID NO. 22: CCATGGTACCCGGGAGCTCGAATTCCGGAGTCTCTTTCAAAACCGTAG
SEQ ID NO. 23: CCTCGCGTCGGGCGATATCGGATCCAAAATCATGTAAGCCCATTCCG
SEQ ID NO. 24: GTGAGTATGGGAAAACAACGCTCCCTTCTTTCTTGTC
SEQ ID NO. 25: AAGAAGGGAGCGTTGTTTTCCCATACTCACAGGCCG
SEQ ID NO. 26: CCATGGTACCCGGGAGCTCGAATTCTAAAATGAAGGTGGTCCGGGAT
SEQ ID NO. 27 (*Pfk*): 
ATGAAGCGAATTGGAGTATTGACAAGCGGCGGCGATGCACCGGGGATGAA
TGCGGCGGTCCGCGCGATTGCCCGTAAAGGGATTTATCACGGCCTGGAAGT
TTACGGCATTCGCCAAGGTTATAACGGATTGATTCAAGGAAACATCCAAAA
GCTCGAAGCAGGATCTGTTGGCGATATTCTCCAGCGGGGCGGCACGGTTTT
GCAGTCGGCAAGAAGCGAAGAATTCAAAACGCCGGAAGGGCAGCAAAAA
GCGATCAGGCAGCTGAAAGACCATGGCATTGAAGCGCTCGTTGTGATCGG
CGGCGACGGTTCCTACCAAGGGGCCAAAAAGTTGACGGAACAGGGCTTTA
ACTGCATTGGTGTGCCAGGGACAATCGATAACGACATCCCGGGGACGGATT
 
TTACAATCGGTTTTGATACGGCATTGAACACAGTGCTTGATGCGATTGATAA
AATTCGCGACACCGCTTCTTCCCACGAACGCACCTTTATTATTGAAGTCATG
GGCAGAAATGCCGGGGATATCGCGCTCTGGTCCGGCCTGGCCGGCGGAGC
CGAATCGATTATTATTCCGGAAGAAAAATATGACTTAAAAGATGTCGTGGAG
CGTCTTGAACAGGGGAGAAAACGCGGCAAACGCCACAGCATCATCATTGT
CGCGGAAGGCGTGATGAGCGGCAACGAGTTTGCTGAACAATTGAAAAAAA
CCGGTGTGATCGGCGATACCCGCGTTTCTGTTCTCGGCCATATCCAGCGCGG
CGGTTCTCCGACGGCATTTGACCGCGTGCTTGCAAGCCGCCTCGGCGCAA
GGGCTGTTGAACTGCTGCTTGAAGGAAAAGGGGGCCGCGCTGTCGGCATT
CAAAATAACCAGCTGGTTGACCACGATATCCTTGAGATTCTCGGAAAACCG
CACGCCGTTAATAAAAACATGTACAAGCTGTCGAAAGAATTGTCGATCTAA
SEQ ID NO. 28 (*Pyk*): 
ATGAAAAAAACCAAAATTGTATGTACAATCGGACCTGCCAGTGAAAGTGTG
GAAATGCTTGAAAGATTAATGGCAAACGGGATGGATGTTTGCCGCCTGAAC
TTCTCGCACGGCAGCCATGAGGAACATCTTGCCCGGATTAAAAATATCCGT
GAAGCTGCAAAAAACCAAAACAAAACGATCGGGCTTCTGCTCGATACAAA
GGGCCCGGAAATCCGCACCCATGATATGAAAGACGGCGGATTCGAGCTCGT
TGAAGGCATGACACCGGTCATTTCAATGACAGAAGTGCTCGGGACACCGG
AAAAATTTTCGGTCACATATGAAGGGCTGATTGATGATGTGCACGTTGGCTC
TAAAATTTTACTTGATGACGGTTTGATTGAACTGGAAGTGACGGCCATCGAT
AAAAACGCCGGTGAAATCCATACAAAAGTGCTGAACCGCGGCGTTTTGAA
AAACAAAAAAGGTGTTAACGTCCCGGGTGTTTCCGTGAACCTTCCGGGCA
TCACCGAAAAAGACGTGAGCGATATCCTGTTCGGGCTTGAACAAGGCATTG
ACTTCATTGCGGCTTCGTTTGTACGCCGGCCGTCCGACGTTTTGGAAATCC
GCCAGCTCCTTGAAGAACACGATGCTTTGCATGTGAAAATTTTCCCTAAAA
TTGAAAACCAGGAAGGCGTCGACAATATCGATGAAATCCTTGCGGTATCAG
ACGGCTTAATGGTTGCCCGCGGCGACCTCGGCGTTGAAATTCCGACCGAAG
CGGTGCCGCTCGTACAAAAAGAAATGATCAGAAAATGTAATACGCTCGGCA
AACCGGTGATTACCGCAACGCAAATGCTTGATTCGATGCAACGCAACCCGC
GCCCGACCCGCGCGGAAGCAAGCGACGTGGCCAACGCCATTTTTGACGGC
ACGGATGCGATCATGCTTTCCGGCGAAACGGCAGCCGGGAAATATCCTGCT
GAAGCGGTTAAGACGATGTACAATATTGCGGTTCATGTGGAAAAAGCAATT
AACCATCGCGATATTCTGAACAAGCGCAGCAAGAGCACGGACCATAATATG
 
ACAGACGCTATCTGCCAGTCCGTTGCCCATACGGCTTTAAATCTTGATGTGA
ATGCCATTATTGCGCCGACTGAAAGCGGCTATACGGCACGCATGATCTCCAA
ATACCGCCCGGCGGCCCCAATCATTGCTGTCACGAGCGATCCGAAAGTACA
ACGCGGCTTAACTGTTGTGTCCGGCGTATACCCACAATTGGGCACAAAGGC
AAACAATACGGATGAAATGCTTGAAATTGCAGTGGAGGAAGCGTTGAAAT
CCGAAATCGTCCATCACGGCGACCTTGTGATCATTACAGCAGGCGTCCCGG
TTGGTGGGAAAGGCACCACCAACCTGATGAAAGTGCACCTGATCGGTGATA
TATTGGCAAAAGGCCAGGGAATCGGCAGAAAATCGGCATTCGGCCCGGTC
ATCGTTGCTGAAAGCCCTGAAGAAGCAAACGCAAAGGCAACAGAAGGTT
GTGTGCTCGTCACGAGAACGACCGACAAAGAAATCATGCCGGCCATTGAA
AAATGCGCCGCGCTGATTACGGAAGAAGGCGGCTTGACAAGCCATGCTGC
AGTTGTTGGCATCAATGTCGGCATTCCGGTCATTGTTGGCGTTGAAAAAGC
CGTTTCCATTTTTGAAGACGGGCAGGAAGTTACGGTAGATGCGGCAACCGG
CTCGGTTTACAACGGCCATGCGACTGTATTGTAA
SEQ ID NO. 29 (*Alr1*): 
ATGATGAGCTTAAAACCATTCTATAGAAAGACATGGGCCGAAATCGATTTAA
CGGCTTTAAAAGAAAACGTCCGCAATATGAAGCGGCACATCGGCGAGCAT
GTCCGCCTGATGGCCGTCGTTAAAGCGAATGCCTACGGACACGGGGATGCA
CAGGTAGCGAAGGCGGCTCTTGCAGAAGGGGCGTCCATTCTTGCTGTGGC
TTTATTGGATGAAGCGCTTTCGCTGAGGGCGCAGGGGATTGAAGAACCGAT
TCTTGTCCTCGGTGCAGTGCCGACCGAATATGCAAGCATTGCCGCGGAAAA
GCGCATTATCGTGACTGGCTACTCCGTCGGCTGGCTGAAAGACGTGCTCGG
TTTTCTGAATGAGGCCGAAGCTCCTCTTGAATATCATTTGAAGATCGACACG
GGCATGGGCCGCCTTGGCTGCAAAACGGAAGAAGAGATCAAAGAAATGAT
GGAGATGACCGAATCGAACGATAAGCTCAATTGTACGGGCGTGTTCACTCA
TTTCGCCACGGCGGACGAAAAGGACACCGATTATTTCAACATGCAGCTTGA
CCGCTTTAAAGAGCTGATCAGCCCCCTCCCGCTTGACCGTTTGATGGTGCA
TTCGTCAAACAGCGCCGCGGGTCTGCGCTTCAGGGAACAGCTATTTAATGC
CGTCCGCTTCGGCATCGGCATGTACGGTTTGGCGCCGTCAACCGAAATAAA
AGACGAGCTGCCGTTTCGTCTGCGGGAAGTGTTTTCGCTTCATACCGAACT
CACCCATGTGAAAAAAATTAAAAAAGGCGAGAGCGTCAGCTACGGGGCGA
CATATACAGCTCAGCGCGACGAATGGATCGGGACAGTCCCCGTCGGGTATG
CCGACGGATGGCTGAGGCGCCTGGCCGGAACGGAAGTGCTGATCGACGGA
 
AAACGCCAAAAAATAGCAGGGAGAATCTGCATGGACCAGTTCATGATTTCC
CTTGCCGAAGAATACCCTGTCGGCACAAAGGTTACCTTGATCGGAAAGCA
AAAAGACGAATGGATCTCAGTCGACGAAATCGCCCAAAATTTGCAGACGA
TCAATTATGAAATTACCTGTATGATAAGTTCAAGGGTGCCCCGTATGTTTTTG
GAAAATGGGAGTATAATGGAAATAAGGAATCCGATCTTGCCTGATCAATCCT
GA
SEQ ID NO. 30 (*Alr2*): 
ATGAAAAAGCTTTGCCGTGAAGTTTGGGTAGAGGTAAATCTTGATGCGATC
AAAAAAAATTTGCGCGCGTTTCGGCGGCATATTCCGAAAAAGAGCAAAATT
ATGGCTGTCGTAAAAGCGAATGCTTATGGTCACGGATCGGTGGAAGTTGCA
CGCCATGCACTTGAACATGGTGCGAGTGAGCTCGCCGTTGCCTCGGTGGA
GGAAGCGGTCGTTTTACGAAAAGCGGGGATTAAAGCGCCGATCCTTGTGCT
TGGTTTCACCCCGCTGAGCTGTGTGAAAGAAGCGGCAGCTTGGAATATATC
GTTATCAGCTTTTCAAGTTGACTGGATTAAAGAAGCGAACGAGATATTGGA
AAATGAAGCAGATCCTAACCGGCTGGCTGTTCATATCAATGTGGATACCGG
CATGGGGCGTTTAGGTGTACGAACAAAGGAAAAGCTTTTAGCAATCGTGG
AAGCGCTGACGGCAAGTGAAAACCTCGAATGGGAAGGAATTTTTACGCAT
TTTTCCACAGCTGACGAACCGGATACTGAGCTAACCATGATTCAACACGAA
AAGTTTATCAGCTTTCTTCGCTTTCTGAAAGAACAAGGCTTTAAGCTGCCTA
CGGTGCATATGAACAATACGGCCGCGGCGATCGCTTTTCCGGAATTCAGCG
CTGATATGATTCGCTTAGGCATCGGAATGTATGGATTATATCCTTCCGATTATA
TCAGGCAGCTTAATCTCGTTAAGCTTGTGCCTGCACTAAGCTTGAAGGCGC
GAATCGCTTATGTGAAAACCATGTTGACTGAACCGCGGACGGTTAGTTATG
GTGCTACATATGTTGCAGAGCGCGGGGAAGTCATTGCCACAATTCCGGTCG
GCTATGCTGACGGCTATTCCCGTGAACTTTCCAACCGCGGTTTTATTCTTCA
TCGAGGAAGACGAGTGCCGGTGGCGGGAAGAGTAACAATGGATATGATAA
TGGTCAGTCTGGGAGAGGGTGAAGGTAAACAAGGAGAGGAAGTCGTGATT
TACGGCCGGCAAAAGGGAGCAGAGATATCTGTTGATGAAATTGCGGAAAT
GCTTGATACGATCAACTATGAAGTGGTATCTACCATAAGCTGGCGCGTCCCT
CGTTTTTATATAAGAGACGGCGAGATTTTTAAAAAGTCGACCCCGCTGTTAT
ACGTGTAG
SEQ ID NO. 31 (*No):* 
ATGAAAGTAATTTTTTTTTCTATGCACCCGTATGAAGAGGAATTTCTGGGTC
CGATTCTGCCGTCTGACTGGGACGTAGAAATGACCCCGGACTTTCTGGACG
AAACCACCGTGGAAAAGGCTAAAGGTGCCCAGGTAGTAAGCCTGTTTGTT
TCTGACAAAGCTGATGGTCCGGTACTGGAAGCGCTGCATTCTTACGGTGTG
GGCCTGCTGGCCCTGCGTTCTGCTGGCTATGATCACATCGATATTGAGACCG
CAAAACGCCTGGGTATCAAAGTAGTTAACGTGCCAGCCTATTCTCCGCACG
CTATCGCTGACCATACTCTGGCTATCATGCTGGCTCTGATTCGTCGTCTGCA
CCGTGCCCATGATAAAGTGCGCCTGGGTGATTTTGATCTGGATGGTCTGATG
GGCTTTGATCTGAACGGCAAAGTTGCTGGTGTAATTGGTCTGGGTAAAATC
GGTCGCCTGGTAGCTACCCGCCTGAAAGCGTTTGGTTGCAAAGTTCTGGGC
TATGATCCATACATTCAGCCGGAAATCGTAGAAAACGTTGATCTGGATACCC
TGATCACTCAGGCTGATATCATTTCTATTCATTGTCCGCTGACCCGTGAAAA
CTTTCATATGTTTAACGAAGAGACTTTTAAGCGTATGAAACCGGGTGCTATT
CTGGTTAACACCGCGCGTGGTGGTCTGATCGATACCAAGGCCCTGCTGGAG
GCCCTGAAGTCTGGTAAACTGGGCGGCGCAGCCCTGGATGTGTATGAATAT
GAACGTGGCCTGTTTTTTAAAAACCACCAAAAAGAAGGTATCAAAGACCC
GTATCTGGCCCAGCTGCTGGGTCTGGCCAACGTAGTGCTGACCGGTCATCA
GGCCTTTCTGACCCGTGAGGCTGTAAAAAACATCGAAGAAACTACCGTAG
AAAACATTCTGGAATGGCAAAAGAACCCGCAGGCAAAGCTGAAAAACGA
AATCTAA
SEQ ID NO. 32 (GSald):

MKIGIPKEIKNNENRVAITPAGVMTLVKAGHDVYVETEAGAGSGFSDSEYEKA
GAVIVPNAEDAWTAEMVLKVKEPLAEEFRYFRPGLILFTYLHLAAAEALTKAL
VEQKVVGIAYETVQLANGSLPLLTPMSEVAGRMSVQVGAQFLEKPHGGKGIL
LGGVPGVRRGKVTIIGGGTAGTNAAKIAVGLGSDVTILDINAERLRELDDLFG
DHVTTLMSNSYHIAECVRESDLVVGAVLIPGAKAPKLVTEEMVRSMTPGSVL
VDIAIDQGGIFETTDRVTTHDDPTYVKHGVVHYAVANMPGAVPRTSTFALTNV
TIPYALQIANKGYRAACLDNPALLKGINTLDGHIVYEAVAAAHNMPYTDVHSL
LHG

In a first aspect of the present invention, there is provided a method of constructing an L-alanine-producing genetically engineered strain, including the steps of:
providing an original strain possessing a pyruvate synthesis pathway;
engineering the genome of the original strain through one, two or three of steps S200, S300 and S400;
S200: introducing overexpressed copies of the *pfk* gene that encodes 6-Phosphofructokinase and of the *pyk* gene that encodes pyruvate kinase;
S300: introducing an overexpressed gene encoding thermostable alanine dehydrogenase (preferably, the overexpressed gene *GSald* that encodes thermostable alanine dehydrogenase);
S400: in case of the genome of the original strain containing an alanine racemase gene, inactivating or deleting the alanine racemase gene.

In some embodiments of the present invention, S200, S300 and S400 may be separately accomplished by:
S200: inserting a copy of the 6-Phosphofructokinase gene *pfk* and a copy of the pyruvate kinase gene *pyk*;
S300: inserting the overexpressed gene *GSald* encoding alanine dehydrogenase which is thermostable at 42 °C to 55 °C;
S400: partially or completely inactivating the alanine racemase gene, or partially or completely deleting the alanine racemase gene, e.g., by knocking out the alanine racemase gene.

An L-alanine-producing genetically engineered strain constructed according to the first aspect of the present invention is capable of production of L-alanine by fermentation under a high-temperature condition of 42 °C or higher (e.g., 42 °C to 55 °C). Step S200 can enhance the glycolysis pathway by enabling overexpression of 6-phosphofructokinase and pyruvate kinase involved in the pathway, thereby providing an increased supply of pyruvate and promoting the production of L-alanine. In step S300, the gene encoding thermostable alanine dehydrogenase is introduced to enhance a pathway that synthesizes L-alanine from pyruvate, effectively increasing the yield of L-alanine. In step S400, the alanine racemase gene can be inactivated or deleted, facilitating the production of optically pure L-alanine. Using any of steps S200, S300, S400 can increase the yield of L-alanine, and the combined use of two or three of them can provide a synergistic effect.

In some embodiments of the present invention, the original strain further possesses a lactate synthesis pathway, and the genome of the original strain contains a lactate dehydrogenase gene. In these cases, the method further includes the step of:
S 100: inactivating or deleting the lactate dehydrogenase gene in the genome of the original strain.

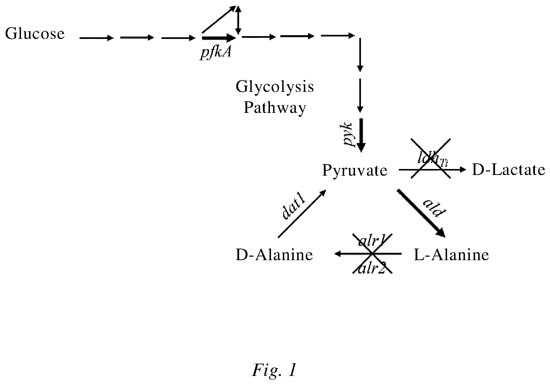

Fig. 1 schematically illustrates alanine synthesis and metabolism pathways in an L-alanine-producing genetically engineered strain according to some embodiments of the present invention. A carbon source is converted into pyruvate through the glycolysis pathway, which then reacts with and consumes NADH and ammonium ions under the catalysis of alanine dehydrogenase, forming L-alanine. Part of the resulting L-alanine is directly secreted extracellularly, and the remainder is converted under the catalysis of alanine racemase into D-alanine, which is then secreted extracellularly. In case the carbon flux downstream of pyruvate is predominated by the lactate synthesis pathway in the original strain, blocking the lactate synthesis pathway can direct the carbon flux to the alanine synthesis pathway as much as possible. Moreover, overexpressing 6-phosphofructokinase and pyruvate kinase that are involved in the glycolysis pathway can enhance the glycolysis pathway and result in an increased supply of pyruvate, thereby promoting the production of alanine. Further, knocking out the alanine racemase gene can block the native D-alanine synthesis pathway, resulting in optically pure L-alanine.

In some embodiments of the present invention, the original strain further possesses a D-lactate synthesis pathway, and its genome contains the *ldh_{Ti}* gene that encodes D-lactate dehydrogenase. In these cases, the method further includes the step of inactivating or deleting the *ldh_{Ti}* gene that encodes D-lactate dehydrogenase in the genome of the original strain (S500). In some preferred embodiments of the present invention, step S500 includes: knocking out the *ldh_{Ti}* gene that encodes D-lactate dehydrogenase in the genome of the original strain.

In some embodiments of the present invention, the *ldh_{Ti}* gene that encodes D-lactate dehydrogenase has a sequence as shown in SEQ ID NO. 31.

In some embodiments of the present invention, the *pfk* gene that encodes 6-phosphofructokinase has a sequence as shown in SEQ ID NO. 27, and the *pyk* gene that encodes pyruvate kinase has a sequence as shown in SEQ ID NO. 28.

In some embodiments of the present invention, the alanine dehydrogenase gene has a sequence as shown in SEQ ID NO. 1.

In some embodiments of the present invention, in step S400, one, two or more types of alanine racemase genes are inactivated or deleted.

In some embodiments of the present invention, the relevant genes are deleted by knockout.

In some embodiments of the present invention, step S400 includes inactivating or deleting the *alr1* or *alr2* gene that encodes alanine racemase. In some embodiments of the present invention, step S400 includes inactivating or deleting the *alr1* and *alr2* genes that encode alanine racemase. In some preferred embodiments of the present invention, step S400 includes knocking out the *alr1* and *alr2* genes that encode alanine racemase.

In some embodiments of the present invention, the *alr1* gene that encodes alanine racemase has a sequence as shown in SEQ ID NO. 29, and the *alr2* gene that encodes alanine racemase has a sequence as shown in SEQ ID NO. 30.

In some embodiments of the present invention, the engineering of the genome of the original strain includes steps S100 and S200. In this way, the lactate synthesis pathway can be blocked and eliminated by inactivating or deleting the lactate dehydrogenase gene, and the glycolysis pathway can be enhanced through overexpression of 6-phosphofructokinase by the *pfk* gene and of pyruvate kinase by the *pyk* gene. Preferably, the engineering of the genome of the original strain further includes step S300. In this way, the yield of L-alanine can be additionally increased by inserting the gene that encodes thermostable alanine dehydrogenase.

In some embodiments of the present invention, the engineering of the genome of the original strain includes steps S100, S200, S300 and S400. In this way, in addition to blocking the lactate synthesis pathway by inactivating or deleting the lactate dehydrogenase gene and to enhancing the glycolysis pathway and thereby providing an increased supply of pyruvate through introducing the genes that overexpress 6-phosphofructokinase and pyruvate kinase to the original strain, the introduced overexpressed alanine dehydrogenase gene can enhance the synthesis pathway of L-alanine from pyruvate, and the alanine racemase gene in the original strain is inactivated or deleted. As a result of these synergistic strategies, high-yield production of optically pure L-alanine can be achieved.

In some embodiments of the present invention, the engineering of the genome of the original strain includes steps S500 and S200. In this way, the lactate synthesis pathway is blocked by inactivating or deleting the *ldh_{Ti}* gene that encodes D-lactate dehydrogenase, and the glycolysis pathway is enhanced by overexpressing the *pfk* and *pyk* genes that encode 6-phosphofructokinase and pyruvate kinase, respectively.

In a preferred example of the present invention, the method includes the steps of:
S500: completely inactivating or completely deleting a lactate dehydrogenase gene in the genome of the original strain, preferably the *ldh_{Ti}* gene that encodes D-lactate dehydrogenase (thereby blocking the lactate synthesis pathway);
S200: overexpressing copies of the *pfk* gene that encodes 6-phosphofructokinase and of the *pyk* gene that encodes pyruvate kinase (thereby enhancing the glycolysis pathway and providing an increased supply of pyruvate);
S300: overexpressing the heterologous *GSald* gene that encodes alanine dehydrogenase (thereby enhancing the synthesis pathway from pyruvate to L-alanine); and
S400: completely inactivating or completely deleting the *alr1* and *alr2* genes that encode alanine racemase (thereby blocking the conversion of L-alanine to D-alanine and facilitating high-yield production of optically pure L-alanine).

In a preferred example of the present invention, the method includes the steps of:
S500: knocking out a lactate dehydrogenase gene in the genome of the original strain, preferably the *ldh_{Ti}* gene that encodes D-lactate dehydrogenase (thereby blocking the lactate synthesis pathway), wherein more preferably, the *ldh_{Ti}* gene has a sequence as shown in SEQ ID NO. 31;
S200: inserting a copy of the *pfk* gene that encodes 6-phosphofructokinase and a copy of the *pyk* gene that encodes pyruvate kinase (thereby enabling overexpression of the *pfk* gene that encodes 6-phosphofructokinase and the *pyk* gene that encodes pyruvate kinase in the original strain, enhancing the glycolysis pathway and providing an increased supply of pyruvate), wherein more preferably, the *pyk* gene that encodes pyruvate kinase has a sequence as shown in SEQ ID NO. 27 and the *pyk* gene that encodes pyruvate kinase has a sequence as shown in SEQ ID NO. 28;
S300: inserting the heterologous *GSald* gene that encodes alanine dehydrogenase (thereby enabling overexpression of the heterologous *GSald* gene that encodes alanine dehydrogenase and enhancing the synthesis pathway from pyruvate to L-alanine), wherein more preferably, the *GSald* gene that encodes alanine dehydrogenase has a sequence as shown in SEQ ID NO. 1; and
S400: knocking out the *alr1* and *alr2* genes that encode alanine racemase (thereby blocking the conversion of L-alanine to D-alanine and facilitating the production of optically pure L-alanine), wherein more preferably, the *alr1* gene that encodes alanine racemase has a sequence as shown in SEQ ID NO. 29, and the *alr2* gene that encodes alanine racemase has a sequence as shown in SEQ ID NO. 30.

It would be appreciated that, unless otherwise specified, the features of the embodiments of the present invention are preferred to be independent, but can be combined as appropriate.

In some embodiments of the present invention, in steps S200 and S300, each of the relevant genes is inserted by adding a copy or copies (i.e., one or more copies) of the gene to the chromosome and ligating in series a promoter upstream thereof.

In some embodiments of the present invention, the promoter is any one of Pₐₗₛ, Plac, Ptrc, Ptac and Pc P43.

In some embodiments of the present invention, the promoter is Pₐₗₛ.

In some embodiments of the present invention, the promoter has a sequence as shown in SEQ ID NO. 2.

In some embodiments of the present invention, the original strain is a thermophilic strain.

In some preferred embodiments of the present invention, the original strain is a *Bacillus* strain.

In some preferred embodiments of the present invention, the original strain is *Bacillus licheniformis,* which is a facultative anaerobic, Gram-positive, endospore-forming bacterium with a fast growth rate, a wide glucose spectrum and capabilities of high-temperature fermentation under a condition of 50 °C. Moreover, it allows for stable genetic manipulation and has been recognized by the US Food and Drug Administration as a "generally regarded as safe" (GRAS) bacterium. Thus, it is promising for providing an ideal platform strain.

In some preferred embodiments of the present invention, the original strain is *Bacillus licheniformis*, *Bacillus coagulans*, *Bacillus methylotrophicus*, thermophilic *Bacillus inulinus* or *Geobacillus stearothermophilus.*

In some preferred embodiments of the present invention, the original strain is *Bacillus licheniformis* ATCC 14580 or a derivative thereof. Examples of the derivative include, but are not limited to, *Bacillus licheniformis* MW3, *Bacillus licheniformis* BN11, etc.

In some preferred embodiments of the present invention, the original strain is *Bacillus licheniformis* BN11, deposited in the China Center for Type Culture Collection on January 8, 2016 as CCTCC NO: M2016026.

In some preferred embodiments of the present invention, the original strain is *Bacillus licheniformis* BN11, wherein the *pfk* gene that encodes 6-phosphofructokinase has a sequence as shown in SEQ ID NO. 27, and the *pyk* gene that encodes pyruvate kinase has a sequence as shown in SEQ ID NO. 28.

In some preferred embodiments of the present invention, the original strain is *Bacillus licheniformis* BN11, wherein the *GSald* gene that encodes alanine dehydrogenase has a sequence as shown in SEQ ID NO. 1.

In some preferred embodiments of the present invention, the original strain is *Bacillus licheniformis* BN11, wherein the endogenous alanine racemase gene includes the *alr1* and *alr2* genes, which have sequences as shown in SEQ ID NOs. 29 and 30, respectively.

In a second aspect of the present invention, there is provided a genetically engineered strain constructed in accordance with the first aspect of the present invention. The genetically engineered strain is capable of production of L-alanine by fermentation at 42 °C or higher (e.g., 42 °C to 55 °C) with a significantly increased yield. It is also capable of high-yield production of optically pure L-alanine.

In a third aspect of the present invention, there is provided use of a genetically engineered strain according to the second aspect of the present invention for L-alanine production. It can be used for production of L-alanine by fermentation with a significantly increased yield under a high-temperature condition. It can be also used for high-yield production of optically pure L-alanine under a high-temperature condition.

In a fourth aspect of the present invention, there is provided a method of producing L-alanine, in which a genetically engineered strain according to the second aspect of the present invention is subject to fermentation culture at a temperature of 42 °C to 55 °C. Examples of the temperature include, but are not limited to, 42 °C, 43 °C, 44 °C, 45 °C, 46 °C, 47 °C, 48 °C, 49 °C, 50 °C, 51 °C, 52 °C, 53 °C, 54 °C and 55 °C.

In some embodiments of the present invention, the fermentation culture step includes: inoculating activated seeds into fermentation culture medium containing a carbon source; and carrying out fed-batch fermentation under a condition at a fermentation culture temperature of 42 °C to 55 °C and an agitation speed of 50 rpm to 350rpm until a predetermined broth density is achieved. Moreover, an inoculum volume of the activated seeds, measured in percentage by volume, may be 3% to 5%, for example, such as 3%, 4% or 5%.

In some embodiments of the present invention, the method further includes, prior to the fermentation culture, seed culture for obtaining the activated seeds, which are then inoculated into the fermentation culture medium and undergoes the fermentation culture.

In some embodiments of the present invention, the seed culture step includes inoculating the genetically engineered strain into seed culture medium and culturing it for 12 h to 16 h under a seed culture temperature at a temperature of 42 °C to 55 °C, resulting in a seed culture solution, i.e., the activated seeds.

In some embodiments of the present invention, the seed culture medium contains the following components: peptone, yeast powder and sodium chloride.

In some embodiments of the present invention, the seed culture medium is composed of the following components: peptone, yeast powder and sodium chloride.

In some embodiments of the present invention, the method includes the steps of:
Seed Culture: inoculating the genetically engineered strain into seed culture medium and culturing it under a condition at a seed culture temperature of 42 °C to 55 °C, resulting in activated seeds; and
Fermentation Culture: inoculating the activated seeds into fermentation culture medium containing a carbon source and carrying out fed-batch fermentation under a condition at a fermentation culture temperature of 42 °C to 55 °C until a predetermined broth density is achieved, wherein an inoculum volume, measured in percentage by volume, may be 3% to 5%.

In some embodiments of the present invention, the method includes the steps of:
Seed Culture: inoculating the genetically engineered strain into seed culture medium and culturing it for 12 h to 16 h under a condition at a seed culture temperature of 42 °C to 55 °C, resulting in activated seeds; and
Fermentation Culture: inoculating the activated seeds into fermentation culture medium containing a carbon source and carrying out fed-batch fermentation under a condition at a fermentation culture temperature of 42 °C to 55 °C and an agitation speed of 50 rpm to 350 rpm until a predetermined broth density is achieved, wherein an inoculum volume, measured in percentage by volume, may be 3% to 5%.

In some embodiments of the present invention, in the fermentation culture step, the carbon source is glucose, glycerol, xylose or arabinose.

In some embodiments of the present invention, in the fermentation culture step, the fermentation culture medium contains the following components: glucose, yeast powder, ammonium sulfate, dipotassium hydrogen phosphate, potassium dihydrogen phosphate, magnesium sulfate, ferrous sulfate and manganese sulfate. In some embodiments of the present invention, the fermentation culture medium is composed of the following components: glucose, yeast powder, ammonium sulfate, dipotassium hydrogen phosphate, potassium dihydrogen phosphate, magnesium sulfate, ferrous sulfate and manganese sulfate.

In some embodiments of the present invention, the achievement of the predetermined broth density is determined based on a detected OD₆₀₀ value. For example, the fermentation is stopped when an OD₆₀₀ of 6.5-8.0 is detected.

In some embodiments of the present invention, the fermentation culture step includes: pH adjustment to about 7.0 with ammonia water; fermentation culture under aeration and agitation (first fermentation culture stage) until an OD₆₀₀ value of 6.5-8.0, followed by stopping aeration; and continued fermentation culture at a reduced agitation rate (second fermentation culture stage) until a predetermined broth density is attained, followed by stopping the fermentation. During the fermentation process, the carbon source may be supplemented when its concentration drops to a certain value.

The ammonia water for pH adjustment may have a concentration of 20-30 percent by volume (v/v), such as 20%, 25% or 30%.

Preferably, the aeration is accomplished with air.

The aeration may be conducted at an aeration rate of 0.8 vvm to 1.2 vvm, such as 0.9 vvm, 1.0 vvm, 1.1 vvm, 1.2 vvm or the like.

The fermentation culture is divided into multiple stages. For example, it may be divided into two stages, including a first fermentation culture stage with aeration and a second fermentation culture stage without aeration. The first fermentation culture stage allows fast growth of the strain, and the second fermentation culture stage enables efficient L-alanine production. The first fermentation culture stage is preferred to be with agitation at a speed of 280 rpm to 320 rpm, such as, for example, 280 rpm, 290 rpm, 300 rpm, 310 rpm, 320 rpm or the like. The first fermentation culture stage lasts for a period of time depending on an OD₆₀₀ value, for example, 6 hours to 8 hours, such as 6 h, 6.5 h, 7 h, 7.5 h, or 8 h. The second fermentation culture stage is preferred to be with agitation at a speed of 60 rpm to 100 rpm, such as, for example, 60 rpm, 70 rpm, 80 rpm, 90 rpm, 100 rpm or the like. The second fermentation culture stage lasts for a period of time also depending on an OD₆₀₀ value (e.g., 1.2), for example, 50 hours to 70 hours, such as 50 h, 55 h, 60 h, 65 h or 70 h.

In the fermentation process, the carbon source may be supplemented once, twice or more times.

Regarding the timing of carbon source supplementation during the fermentation process, it may be supplemented, for example, once its concentration drops to 30 g/L to 50 g/L, and again supplemented, for example, when the concentration drops to 20 g/L to 40 g/L. Preferably, the carbon source is glucose.

In some embodiments of the present invention, the carbon source is supplemented only once. In these cases, the carbon source may be supplemented upon the concentration dropping to 30 g/L to 50 g/L (e.g., 30 g/L, 35 g/L, 40 g/L, 45 g/L or 50 g/L), thereby increasing the concentration to 80 g/L to 120 g/L (e.g., 80 g/L, 90 g/L, 100 g/L, 110 g/L or 120 g/L). Preferably, the carbon source is glucose.

In some embodiments of the present invention, the carbon source is supplemented twice: the first supplementation may occur when the concentration drops to 30 g/L to 50 g/L (e.g., 30 g/L, 35 g/L, 40 g/L, 45 g/L or 50 g/L), resulting in a new concentration of 80 g/L to 120 g/L (e.g., 80 g/L, 90 g/L, 100 g/L, 110 g/L or 120 g/L); and the second supplementation may be conducted upon the concentration dropping to 20 g/L to 40 g/L (e.g., 20 g/L, 25 g/L, 30 g/L, 35 g/L or 40 g/L) to raise the concentration back to 60 g/L to 80 g/L (e.g., 60 g/L, 65 g/L, 70 g/L, 75 g/L or 80 g/L). Preferably, the carbon source is glucose.

In some embodiments of the present invention, the fermentation culture step includes: pH adjustment to 7.0±0.2 with 20-30% (v/v) ammonia water; fermentation culture for 6-8 hours under aeration at a rate of 0.8 vvm to 1.2 vvm and under agitation at an initial speed of 280 rpm to 320 rpm so that an OD₆₀₀ value of 6.5-8.0 is achieved, followed by stopping aeration; and continued fermentation culture for 50 hours to 70 hours (e.g., 60 hours) at a modified agitation speed of 60 rpm to 100 rpm, followed by stopping the fermentation. Moreover, during the fermentation process, the carbon source may be supplemented once, twice or more times when its concentration drops to a certain value. In some embodiments, the carbon source is supplemented only once. In these cases, the carbon source may be supplemented upon the concentration dropping to 30 g/L to 50 g/L, increasing the concentration to 80 g/L to 120 g/L. In some other embodiments, the carbon source is supplemented twice. In these cases, the carbon source is supplemented for the first time when its concentration drops to 30 g/L to 50 g/L so that the concentration rises back to 80 g/L to 120 g/L. Subsequently, when the concentration further drops to 20 g/L to 40 g/L, the carbon source is supplemented for a second time to raise the concentration back to 60 g/L to 80 g/L. Preferably, the carbon source is glucose.

In some embodiments of the present invention, the carbon source in the fermentation culture step is glucose, and the fermentation culture step includes: pH adjustment and maintenance to and at 7.0 with 25% ammonia water; fermentation culture for 6 hours to 8 hours under aeration at a rate of 1 vvm and under agitation at an initial speed of 300 rpm so that an OD₆₀₀ value of the *Bacillus licheniformis* strain reaches 6.5-8.0, followed by stopping aeration; and continued fermentation culture for 60 h at a modified agitation speed of 80 rpm, followed by stopping the fermentation. During the fermentation process, glucose is supplemented when its concentration drops to 30 g/L to 50 g/L, thereby increasing the glucose concentration to 100 g/L. When the glucose concentration drops to 20-40 g/L at a later time, glucose supplementation is again conducted to raise the glucose concentration to 70 g/L.

The present invention will be described in greater detail below by way of a few examples, which are intended to be illustrative rather than limiting the scope of the present invention in any sense. These examples are provided in accordance with the teachings of the present invention. Although specific implementation and operation details are set forth, the scope of the invention is in no way limited to the following examples. For any experimental procedure employed in the following examples (e.g., PCR amplification, transformation, gene knockout, gene insertion, etc.), if no particular conditions are specified, it is preferably carried out in the manner and conditions described above. Otherwise, it is generally carried out in a conventional manner and conditions, for example, as taught in Molecular Cloning: A Laboratory Manual by Sambrook et al. (New York: Cold Spring Harbor Laboratory Press, 1989), or under conditions suggested by the manufacturer.

All the materials and reagents used in the following examples are available from commercial sources, unless otherwise specified.

An alanine concentration in each fermentation broth sample was measured by high-performance liquid chromatography (HPLC). Sodium 1-octanesulfonate was weighed at 0.54 g and added to 800 mL of ultrapure water, and the pH was then adjusted to 2.1 with phosphoric acid. Methanol at 100 mL and ultrapure water were then successively added so that a volume of 1 L was obtained, as a mobile phase. The sample was injected at a volume of 10 µL into a ZORBAX Eclipse XDB-C18 column (150 mm × 4.6 mm, 5 µm) at a temperature of 30 °C and caused to flow through the column at a flow rate of 0.8 mL/min, and the measurement was performed by a DAD detector at a wavelength of 210 nm. Before being measured, the sample was boiled for 10 min and centrifuged at 8000 rpm for 5 min. The supernatant was then taken and diluted an appropriate number of times so that alanine was present therein at a final concentration of 0.2 g/L to 1 g/L.

Optical purity of alanine in each fermentation broth sample was measured by HPLC using a 2 mM aqueous copper sulfate solution as a mobile phase. The sample was injected at a volume of 10 µL into a SUMICHIRAL OA-5000 (150 mm × 4.6 mm, 5 µm) at a temperature of 30 °C and caused to flow through the column at a flow rate of 0.5 mL/min, and the measurement was performed by a DAD detector at a wavelength of 254 nm. Before the measurement, the sample was boiled for 10 min and centrifuged at 8000 rpm for 5 min. The supernatant was then taken and diluted an appropriate number of times so that alanine was present therein at a final concentration of 0.2 g/L to 1 g/L.

Glucose concentrations were measured on an SBA-40D biosensor analyzer (Biology Institute of Shandong Academy of Sciences).

In the following examples, *Bacillus licheniformis* strains were used as original strains to construct genetically engineered strains capable of producing L-alanine under a high-temperature condition. It would be appreciated that, in the following examples, strains of other bacteria (for example, including, but not limited to, *Bacillus coagulans*, *Bacillus methylotrophicus*, thermophilic *Bacillus inulinus*, *Geobacillus stearothermophilus,* etc.) may also be used as original strains to construct genetically engineered strains capable of producing L-alanine under a high-temperature condition, without departing from the scope of the present invention.

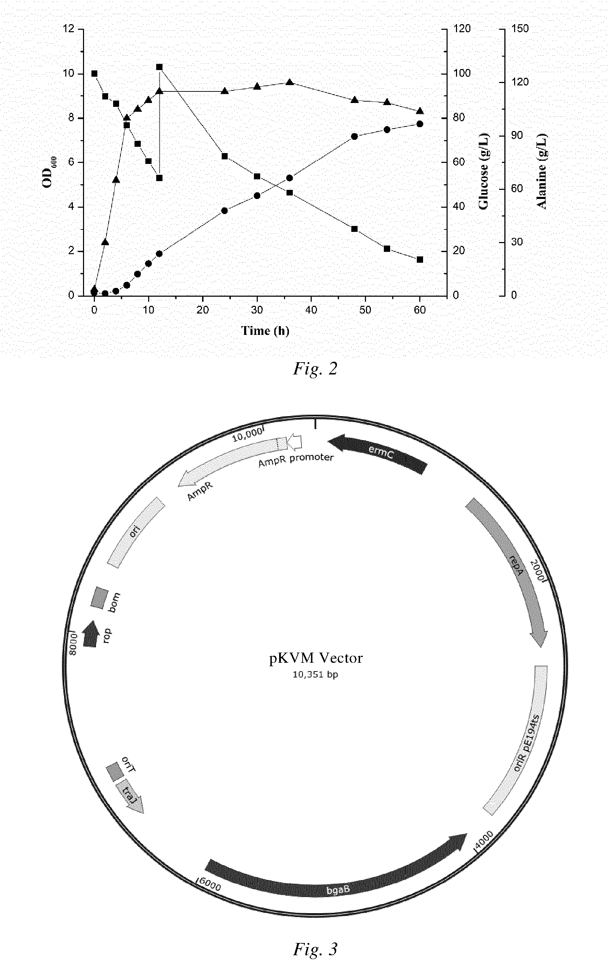

Fig. 3 shows the structure of a pKVM vector, which contains two antibiotic resistance genes: *ampR* (ampicillin resistance) and *ermC* (erythromycin gene), as well as the *bgaB* gene that encodes galactosidase. All of these genes can be used as selectable markers. This plasmid has a heat-sensitive origin of replication (oriT pE194ts) and origins of replication in *Escherichia coli* (ori) and *Bacillus licheniformis* (oriT).

*Bacillus licheniformis* BN11 is a lactate-producing strain constructed from ATCC 14580. It was deposited in the China Center for Type Culture Collection on January 8, 2016 as CCTCC NO: M2016026.

Inoculum volumes are measured in percentage by volume.

Unless otherwise specified, for any newly inserted gene, one copy of it was inserted.

### Example 1: Construction of Bacillus licheniformis Strain with D-Lactate Synthesis Pathway Being Knocked out

1.1 Primers were designed according to the sequence of the *Bacillus licheniformis* ATCC 14580 genome (GenBank No. NC_006270.3). Separately with pKVM-XPDdldh-UpF (SEQ ID NO. 3) and XPDdldh-UpR (SEQ ID NO. 4), as well as, XPDdldh-DownF (SEQ ID NO. 5) and pKVM-XPDdldh-DownR (SEQ ID NO. 6), as upstream and downstream primers, respectively, PCR amplification was performed to obtain fragments containing homology arms upstream and downstream of a promoter for the gene *Idh_{Ti}.*

1.2 The PCR product from step 1.1 was purified, and recombinant PCR amplification was conducted with pKVM-XPDdldh-UpF and pKVM-XPDdldh-DownR as primers. After being purified, the PCR product was cloned into a pKVM vector by seamless cloning, which was then transformed into *Escherichia coli* S17, resulting in S17-pKVMΔ*ldh_{Ti}*.

1.3 Strains of S17-pKVMΔ*ldh_{Ti}* (*Escherichia coli*) obtained in step 1.2 and *Bacillus licheniformis* BN11 (original strain) were separately cultured by fermentation to an OD₆₀₀ value of 1.2. The resulting strains were then centrifuged at 6000 rpm for 5 min and washed twice with PBS. Subsequently, the engineered *Escherichia coli* S17-pKVMΔ*ldh_{Ti}* and the *Bacillus licheniformis* BN11 strains with the same OD₆₀₀ (and hence the same concentration) were mixed up at a volume ratio of 7: 1, and the mixture was added dropwise to LB plates. After overnight culture at 30°C, the strains were diluted and spread on LB plates containing erythromycin and polymyxin, followed by culture at 30°C. The screened transformants were cultured at 37 °C in LB medium containing erythromycin and then transferred to and cultured in LB medium at 50 °C. After that, they are diluted, spread on LB plates containing erythromycin and cultured at 50 °C, in order to screen for single-crossover transformants. The single-crossover transformants were continuously cultured in LB medium at 30°C for two generations, diluted, spread on LB plates and cultured at 37°C overnight. Double-crossover transformants were then selected by reverse screening based on resistance to erythromycin. As verified by PCR using pKVM-XPDdldh-UpF and pKVM-XPDdldh-DownR as primers and sequencing, a genetically engineered strain with a D-lactate synthesis pathway (i.e., the gene *ldh_{Ti}* encoding D-lactate dehydrogenase (SEQ ID NO. 31)) being knocked out was successfully constructed, denoted as BN11Δ*ldh_{Ti}.*

### Example 2: Construction of Bacillus licheniformis Strain Overexpressing 6-Phosphofructokinase and Pyruvate Kinase

2.1 Primers were designed according to the sequence of the *Bacillus licheniformis* ATCC 14580 genome (GenBank No. NC_006270.3). Separately with pKVM-XPDldh-UpF (SEQ ID NO. 7) and Pₐₗₛ-XPDldh-UpR (SEQ ID NO. 8), XPDldh-Pₐₗₛ-F (SEQ ID NO. 9) and PFYAK-Pₐₗₛ-R (SEQ ID NO. 10), Pₐₗₛ-PFYAK-F (SEQ ID NO. 11) and XPDldh-PFYAK-R (SEQ ID NO. 12), as well as PFYAK-XPDldh-DownF (SEQ ID NO. 13) and pKVM-XPDldh-DownR (SEQ ID NO. 14), as upstream and downstream primers, respectively, PCR amplification was performed to obtain fragments containing a homology arm upstream of the *ldh* gene, the Pₐₗₛ promoter (SEQ ID NO. 2), the *pfk* (SEQ ID NO. 27) and *pyk* (SEQ ID NO. 28) gene cluster and a homology arm downstream of the *ldh* gene.

2.2 The PCR product from step 2.1 was purified, and recombinant PCR amplification was conducted with pKVM-XPDldh-UpF and pKVM-XPDldh-DownR as primers. After being purified, the PCR product was cloned into a pKVM vector by seamless cloning, which was then transformed into *Escherichia coli* S17, resulting in S17-pKVM-PFYAK.

2.3 Strains of *Escherichia coli* S17-pKVM-PFYAK obtained in step 2.2 and *Bacillus licheniformis* BN11Δ*ldh_{Ti}* were subjected to conjugation transfer and homologous recombination conducted in the same manner as in Example 1, resulting in a strain overexpressing the *pfk* and *pyk* genes, denoted as BN11Δ*ldh_{Ti}*-PFYAK.

### Example 3: Construction of Strain Overexpressing Heterologous Alanine Dehydrogenase

3.1 Based on the amino acid sequence of alanine dehydrogenase, the BLAST method was used to identify potentially thermostable alanine dehydrogenase (with an amino acid sequence as shown in SEQ ID NO. 32 and a protein sequence number corresponding to WP_033014465.1) in the genome of *Geobacillus stearothermophilus.* This alanine dehydrogenase gene sourced from *Geobacillus stearothermophilus,* named *GSald,* was subject to codon optimization based on the *Bacillus licheniformis* genome (a sequence of the optimized version is as shown in SEQ ID NO. 1) and synthesized together with the Pₐₗₛ promoter (SEQ ID NO. 2) from 5' to 3'. The merged sequence is denoted as Pₐₗₛ*-GSald.*

3.2 Primers were designed according to the sequence of the *Bacillus licheniformis* ATCC 14580 genome (GenBank No. NC_006270.3). Separately with pKVM-XPDdldh-UpF (SEQ ID NO. 3) and Pₐₗₛ-XPDdldh-UpR (SEQ ID NO. 15), XPDdldh-Pₐₗₛ-F (SEQ ID NO. 16) and XPDdldh-GSald-R (SEQ ID NO. 17), as well as GSald-XPDdldh-DownF (SEQ ID NO. 18) and pKVM-XPDdldh-DownR (SEQ ID NO. 6), as upstream and downstream primers, respectively, PCR amplification was conducted to obtain fragments containing a homology arm upstream of a promoter for the *ldh_{Ti}* gene, the Pₐₗₛ*-GSald* gene and a homology arm downstream of the *ldh_{Ti}* gene.

3.3 The PCR product from step 3.2 was purified, and recombinant PCR amplification was conducted with pKVM-XPDdldh-UpF and pKVM-XPDdldh-DownR as primers. After being purified, the PCR product was cloned into a pKVM vector by seamless cloning, which was then transformed into *Escherichia coli* S17, resulting in S17-pKVM-*GSald*. Strains of *Escherichia coli* S17-pKVM-*GSald* and *Bacillus licheniformis* BN11Δ*ldh_{Ti}*-PFYAK were subjected to conjugation transfer and homologous recombination conducted in the same manner as in Example 1, resulting in a strain expressing heterologous *GSald,* denoted as BA-1.

### Example 4: Knockout of Alanine Racemase Genes in Bacillus Licheniformis

4.1 Primers were designed according to the sequence of the *Bacillus licheniformis* ATCC 14580 genome (GenBank No. NC_006270.3). Separately with pKVM-XPDalr1-UpF (SEQ ID NO. 19) and XPDalr1-UpR (SEQ ID NO. 20), as well as, XPDalr1-DownF (SEQ ID NO. 21) and pKVM-XPDalr1-DownR (SEQ ID NO. 22), as upstream and downstream primers, respectively, PCR amplification was performed to obtain fragments containing homology arms upstream and downstream of the gene *alr1.*

4.2 The PCR product from step 4.1 was purified, and recombinant PCR amplification was conducted with pKVM-XPDalrl-UpF and pKVM-XPDalr1-DownR as primers. After being purified, the PCR product was cloned into a pKVM vector by seamless cloning, which was then transformed into *Escherichia coli* S17, resulting in S17-pKVMΔ*alr1*.

4.3 Strains of *Escherichia coli* S17-pKVMΔ*alr1* and *Bacillus licheniformis* BA-1 were subjected to conjugation transfer and homologous recombination conducted in the same manner as in Example 1, resulting in a strain with the *alr1* gene (SEQ ID NO. 29) being knocked out, denoted as BA-1Δ*alr1*.

4.4. Primers were designed according to the sequence of the *Bacillus licheniformis* ATCC 14580 genome (GenBank No. NC_006270.3). Separately with pKVM-XPDalr2-UpF (SEQ ID NO. 23) and XPDalr2-UpR (SEQ ID NO. 24), as well as, XPDalr2-DownF (SEQ ID NO. 25) and pKVM-XPDalr2-DownR (SEQ ID NO. 26), as upstream and downstream primers, respectively, PCR amplification was performed to obtain fragments containing homology arms upstream and downstream of the gene *alr2.*

4.5 The PCR product from step 4.4 was purified, and recombinant PCR amplification was conducted with pKVM-XPDalr2-UpF and pKVM-XPDalr2-DownR as primers. After being purified, the PCR product was cloned into a pKVM vector by seamless cloning, which was then transformed into *Escherichia coli* S17, resulting in S 17-pKVMΔalr2.

4.6 Strains of *Escherichia coli* S17-pKVMΔ*alr2* and *Bacillus licheniformis* BA-1Δalr1 were subjected to conjugation transfer and homologous recombination conducted in the same manner as in Example 1, resulting in a strain with the *alr1* (SEQ ID NO. 29) and *alr2* (SEQ ID NO. 30) genes being both knocked out, denoted as BLA-1.

### Example 5: Fed-Batch Fermentation (in 5-L Fermenter) of Recombinant Bacillus licheniformis BLA-1 at 42 °C

### 5.1 Seed Culture Medium and Fermentation Culture Medium

Seed culture medium composition: LB medium, yeast powder 5 g/L, tryptone 10 g/L, sodium chloride 10 g/L.

Fermentation culture medium composition: glucose 100 g/L, yeast powder 5 g/L, ammonium sulfate 5 g/L, dipotassium hydrogen phosphate trihydrate 1.3 g/L, potassium dihydrogen phosphate 0.5 g/L, magnesium sulfate heptahydrate 0.5 g/L, ferrous sulfate heptahydrate 20 mg/L, manganese sulfate tetrahydrate 20 mg/L. Glucose was separately sterilized at 115 °C for 20 min.

### 5.2 Seed Culture

BLA-1 was transferred from a glycerol tube to a shake tube containing 5 mL of LB medium and incubated at 50 °C for 12 h with shaking at 200 rpm. After that, it was inoculated at an inoculum volume of 3% to 5% into 150 mL of LB medium contained in a 500-mL Erlenmeyer flask and incubated at 50 °C for 12 h with shaking at 200 rpm, resulting in a seed solution (i.e., activated seeds). In this step, seed activation was accomplished.

### 5.3 Fermentation Culture

The seed solution obtained in step 5.2 was inoculated at an inoculum volume of 5% into fermentation culture medium contained in a 5-L fermenter, resulting in a total volume of 3 L. After a fermentation pH was controlled to 7.0 with 25% (v/v) ammonia water, fermentation culture was started at a temperature of 42 °C under aeration (by air) at an aeration rate of 1.0 vvm and agitation at a speed of 300 rpm. After 6-8 hours, *Bacillus licheniformis* was fermented to an OD₆₀₀ of 6.5-8.0. At this time, the aeration was stopped, and the agitation speed was decreased to 80 rpm. The fermentation was then resumed and ended 60 h later. During the fermentation process, when a concentration of glucose (carbon source) dropped to 30 g/L to 50 g/L, glucose was supplemented to increase the concentration to 100 g/L. After 60 hours of fermentation, an L-alanine concentration in the fermentation broth was detected as 93.7g/L, corresponding to a glucose-to-alanine conversion rate of 74.4%, and the optical purity of L-alanine was 98.9%.

### Example 6: Fed-Batch Fermentation (in 5-L Fermenter) of Recombinant Bacillus licheniformis BLA-1 at 50 °C

6.1 Seed Culture Medium and Fermentation Culture Medium
   See 5.1 in Example 5
6.2 Seed Culture
   See 5.2 in Example 5
6.3 Fermentation Culture
   A seed solution obtained in step 6.2 was inoculated at an inoculum volume of 5% into fermentation culture medium contained in a 5-L fermenter, resulting in a total volume of 3 L. After a fermentation pH was controlled to 7.0 with 25% (v/v) ammonia water, fermentation culture was started at a temperature of 50 °C under aeration at an aeration rate of 1.0 vvm and agitation at a speed of 300 rpm. After 6-8 hours, *Bacillus licheniformis* was fermented to an OD₆₀₀ of 6.5-8.0. At this time, the aeration was stopped, and the agitation speed was decreased to 80 rpm. The fermentation was then resumed and ended 60 h later. During the fermentation process, when a concentration of glucose dropped to 30 g/L to 50 g/L, glucose was supplemented to increase the concentration to 100 g/L. Fig. 2 is a plot diagram of this fermentation process. After 60 hours of fermentation, an L-alanine concentration in the fermentation broth was detected as 96.8g/L, corresponding to a glucose-to-alanine conversion rate of 76.8%, and the optical purity of L-alanine was 98.9%.

### Example 7: Fed-Batch Fermentation (in 5-L Fermenter) of Recombinant Bacillus licheniformis BLA-1 at 55 °C

7.1 Seed Culture Medium and Fermentation Culture Medium
   See 5.1 in Example 5
7.2 Seed Culture
   See 5.2 in Example 5
7.3 Fermentation Culture
   A seed solution obtained in step 7.2 was inoculated at an inoculum volume of 5% into fermentation culture medium contained in a 5-L fermenter, resulting in a total volume of 3 L. After a fermentation pH was controlled to 7.0 with 25% (v/v) ammonia water, fermentation culture was started at a temperature of 55 °C under aeration at an aeration rate of 1.0 vvm and agitation at a speed of 300 rpm. After 6-8 hours, *Bacillus licheniformis* was fermented to an OD₆₀₀ of 3.0-4.5. At this time, the aeration was stopped, and the agitation speed was decreased to 80 rpm. The fermentation was then resumed and ended 60 h later. After 60 hours of fermentation, an L-alanine concentration in the fermentation broth was detected as 21.4 g/L, corresponding to a glucose-to-alanine conversion rate of 63.8%, and the optical purity of L-alanine was 97.2%.

### Example 8: Fed-Batch Fermentation (in 50-L Fermenter) of Recombinant Bacillus licheniformis BLA-1 at 42 °C

8.1 Seed Culture Medium and Fermentation Culture Medium
   See 5.1 in Example 5
8.2 Seed Culture
   BLA-1 was transferred from a glycerol tube to a shake tube containing 5 mL of LB medium and incubated at 50 °C for 12 h with shaking at 200 rpm. After that, it was inoculated at an inoculum volume of 3% to 5% into 150 mL of LB medium contained in a 500-mL Erlenmeyer flask and incubated at 50 °C for 12 h with shaking at 200 rpm. Finally, it was further inoculated at an inoculum volume of 3% to 5% into 1.5 L of LB medium contained in a 5-L Erlenmeyer flask and incubated at 50 °C for 12 h with shaking at 200 rpm.
8.3 Fermentation Culture
   The seed solution obtained in step 8.2 was inoculated at an inoculum volume of 5% into fermentation culture medium contained in a 50-L fermenter, resulting in a total volume of 30 L. After a fermentation pH was controlled to 7.0 with 25% (v/v) ammonia water, fermentation culture was started at a temperature of 42 °C under aeration at an aeration rate of 1.0 vvm and agitation at a speed of 300 rpm. After 6-8 hours, *Bacillus licheniformis* was fermented to an OD₆₀₀ of 6.5-8.0. At this time, the aeration was stopped, and the agitation speed was decreased to 80 rpm. The fermentation was then resumed and ended 60 h later. During the fermentation process, when a concentration of glucose dropped to 30 g/L to 50 g/L, glucose was supplemented to increase the concentration to 100 g/L. After 60 hours of fermentation, an L-alanine concentration in the fermentation broth was detected as 40.5g/L, corresponding to a glucose-to-alanine conversion rate of 70.7%, and the optical purity of L-alanine was 96.6%.

### Example 9: Fed-Batch Fermentation (in 50-L Fermenter) of Recombinant Bacillus licheniformis BLA-1 at 50 °C

9.1 Seed Culture Medium and Fermentation Culture Medium
   See 5.1 in Example 5
9.2 Seed Culture
   See 8.2 in Example 8
9.3 Fermentation Culture
   A seed solution obtained in step 9.2 was inoculated at an inoculum volume of 5% into fermentation culture medium contained in a 50-L fermenter, resulting in a total volume of 30 L. After a fermentation pH was controlled to 7.0 with 25% (v/v) ammonia water, fermentation culture was started at a temperature of 50 °C under aeration at an aeration rate of 1.0 vvm and agitation at a speed of 300 rpm. After 6-8 hours, *Bacillus licheniformis* was fermented to an OD₆₀₀ of 6.5-8.0. At this time, the aeration was stopped, and the agitation speed was decreased to 80 rpm. The fermentation was then resumed and ended 60 h later. During the fermentation process, when a concentration of glucose dropped to 30 g/L to 50 g/L, glucose was supplemented to increase the concentration to 100 g/L. After 60 hours of fermentation, an L-alanine concentration in the fermentation broth was detected as 108.2 g/L, corresponding to a glucose-to-alanine conversion rate of 73.3%, and the optical purity of L-alanine was 99.1%.

### Example 10: Fed-Batch Fermentation (in 50-L Fermenter) of Recombinant Bacillus licheniformis BLA-1 at 55 °C

### 10.1 Seed Culture Medium and Fermentation Culture Medium

See 5.1 in Example 5

### 10.2 Seed Culture

See 8.2 in Example 8

### 10.3 Fermentation Culture

A seed solution obtained in step 10.2 was inoculated at an inoculum volume of 5% into fermentation culture medium contained in a 50-L fermenter, resulting in a total volume of 30 L. After a fermentation pH was controlled to 7.0 with 25% (v/v) ammonia water, fermentation culture was started at a temperature of 55 °C under aeration at an aeration rate of 1.0 vvm and agitation at a speed of 300 rpm. After 6-8 hours, *Bacillus licheniformis* was fermented to an OD₆₀₀ of 4.0-6.0. At this time, the aeration was stopped, and the agitation speed was decreased to 80 rpm. The fermentation was then resumed and ended 60 h later. After 60 hours of fermentation, an L-alanine concentration in the fermentation broth was detected as 26.6 g/L, corresponding to a glucose-to-alanine conversion rate of 59.0%, and the optical purity of L-alanine was 98.7%.

### Example 11: Fermentation Culture Results of BN11Δldh_{Ti}-PFYAK Strain Constructed in Example 2

Fermentation culture of the BN11Δ*ldh_{Ti}*-PFYAK strain obtained in Example 2 was carried out in accordance with the method and experimental parameters of Example 6.

As a result, a mixture of L- and D-alanine was produced at a yield of 64 g/L, and the optical purity of L-alanine was only 67.7% and therefore unsatisfactory (the optical purity was typically required to be 98% or above).

### Example 12: Fermentation Culture Results of BA-1 Strain Constructed in Example 3

Fermentation culture of the BA-1 strain obtained in Example 3 was carried out in accordance with the method and experimental parameters of Example 6.

As a result, a mixture of L- and D-alanine was produced at a yield of 129 g/L, and the optical purity of L-alanine was only 70.8% and therefore unsatisfactory.

The results of Examples 11 and 12 show that knockout of the alanine racemase genes of *Bacillus licheniformis* is necessary for the achievement of high optical purity of L-alanine.

### Example 13: Knockout of Only One Alanine Racemase Gene

Fermentation culture of a strain with either of the *alr1* (SEQ ID NO. 29) and *alr2* (SEQ ID NO. 30) genes encoding alanine racemase being knocked out using the method of Example 4 was carried out in accordance with the method of Example 6.

As a result, it was found to be unsatisfactory in terms of optical purity.

Preferred specific embodiments of the present invention have been described in detail above. It is to be understood that, those of ordinary skill in the art can make various modifications and changes based on the concept of the present invention without exerting any creative effort. Accordingly, all the technical solutions that can be obtained by those skilled in the art by logical analysis, inference or limited experimentation in accordance with the concept of the present invention on the basis of the prior art are intended to fall within the protection scope as defined by the claims.

## Claims

1. A method of constructing an L-alanine-producing genetically engineered strain, **characterized in** comprising steps of:
providing an original strain possessing a pyruvate synthesis pathway;
engineering a genome of the original strain through any one step, any two steps or three steps of S200, S300 and S400;
S200: inserting a copy of a 6-Phosphofructokinase gene *pfk* and a copy of a pyruvate kinase gene *pyk*;
S300: inserting a gene *GSald* for alanine dehydrogenase thermostable at 42 °C to 55 °C;
S400: inactivating or deleting an alanine racemase gene contained in a genome of the original strain.

2. The method of constructing an L-alanine-producing genetically engineered strain of claim 1, **characterized in that** the original strain further possesses a lactate synthesis pathway and a genome of the original strain contains a lactate dehydrogenase gene; the method of constructing further comprises step of:
S100: inactivating or deleting a lactate dehydrogenase gene in a genome of the original strain.

3. The method of constructing an L-alanine-producing genetically engineered strain of claim 2, wherein the original strain further possesses a D-lactate synthesis pathway and a genome of the original strain contains a D-lactate dehydrogenase gene *ldh_{Ti}*;
the method of constructing further comprises step of S500: inactivating or deleting a D-lactate dehydrogenase gene *ldh_{Ti}* contained in a genome of the original strain;
preferably, a sequence of the D-lactate dehydrogenase gene *ldh_{Ti}* is as shown in SEQ ID NO. 31.

4. The method of constructing an L-alanine-producing genetically engineered strain of claim 1, **characterized in that** a sequence of the 6-phosphofructokinase gene *pfk* is as shown in SEQ ID NO. 27, a sequence of the pyruvate kinase gene *pyk* is as shown in SEQ ID NO. 28; and/or
a sequence of the *GSald* gene for alanine dehydrogenase is as shown in SEQ ID NO. 1.

5. The method of constructing an L-alanine-producing genetically engineered strain of claim 1, **characterized in that**, in step S400, one, two or more types of alanine racemase genes are inactivated or deleted; and/or
the step S400 comprises inactivating or deleting an alanine racemase gene *alr1* and an alanine racemase gene *alr2;*
preferably, a sequence of the alanine racemase gene *alr1* is as shown in SEQ ID NO. 29, and a sequence of the alanine racemase gene *alr2* is as shown in SEQ ID NO. 30.

6. The method of constructing an L-alanine-producing genetically engineered strain of claim 2, **characterized in that** engineering of a genome of the original strain comprises step S100 and step S200;
preferably, engineering of a genome of the original strain comprises step S100, step S200 and step S300.

7. The method of constructing an L-alanine-producing genetically engineered strain of claim 2, **characterized in that** engineering of a genome of the original strain comprises step S100, step S200, step S300 and step S400.

8. The method of constructing an L-alanine-producing genetically engineered strain of claim 3, **characterized in** comprising steps of:
S500: knocking out a D-lactate dehydrogenase gene *ldh_{Ti}* contained in a genome of the original strain;
S200: inserting a copy of a 6-Phosphofructokinase gene *pfk* and a copy of a pyruvate kinase gene *pyk*;
S300: inserting a heterologous alanine dehydrogenase gene *GSald*; and
S400: knocking out an alanine racemase gene *alr1* and an alanine racemase gene *alr2.*

9. The method of constructing an L-alanine-producing genetically engineered strain of claim 1, **characterized in that**, in step S200 and step S300, relevant genes are inserted by adding their copies to a chromosome and ligating promoters in series upstream thereof.

10. The method of constructing an L-alanine-producing genetically engineered strain of claim 9, **characterized in that** the promoter is Pₐₗₛ.

11. The method of constructing an L-alanine-producing genetically engineered strain of claim 10, **characterized in that** a sequence of the promoter is as shown in SEQ ID NO. 2.

12. The method of constructing an L-alanine-producing genetically engineered strain of any of claims 1 to 11, **characterized in that** the L-alanine-producing genetically engineered strain is capable of producing L-alanine by fermentation at 42 °C to 55 °C.

13. The method of constructing an L-alanine-producing genetically engineered strain of any of claims 1 to 11, **characterized in that** the original strain is a thermophilic strain.

14. The method of constructing an L-alanine-producing genetically engineered strain of any of claims 1 to 11, **characterized in that** the original strain is *Bacillus.*

15. The method of constructing an L-alanine-producing genetically engineered strain of claim 14, **characterized in that** the original strain is *Bacillus licheniformis*, *Bacillus coagulans*, *Bacillus methylotrophicus*, thermophilic *Bacillus inulinus* or *Geobacillus stearothermophilus.*

16. The method of constructing an L-alanine-producing genetically engineered strain of claim 14, **characterized in that** the original strain is *Bacillus licheniformis* ATCC 14580 or a derivative thereof.

17. The method of constructing an L-alanine-producing genetically engineered strain of claim 14, **characterized in that** the original strain is *Bacillus licheniformis* BN11, deposited in the China Center for Type Culture Collection on January 8, 2016 as CCTCC NO: M2016026.

18. A genetically engineered strain constructed according to the method of any of claims 1 to 17.

19. Use of the genetically engineered strain of claim 18 for L-alanine production.

20. A method of producing L-alanine, in which the genetically engineered strain of claim 18 is subjected to fermentation culture, wherein fermentation culture is at a temperature of 42 °C to 55 °C

21. The method of producing of claim 20, **characterized in that** the fermentation culture step comprises: inoculation of activated seeds into fermentation culture medium containing a carbon source and fed-batch fermentation at an agitation speed of 50 rpm to 350 rpm to a predetermined broth density.

22. The method of producing of claim 21, **characterized in that**, in the fermentation culture step, the carbon source is glucose, glycerol, xylose or arabinose.

23. The method of producing of claim 21, **characterized in that**, in the fermentation culture step, the fermentation culture medium contains following components: glucose, yeast powder, ammonium sulfate, dipotassium hydrogen phosphate, potassium dihydrogen phosphate, magnesium sulfate, ferrous sulfate and manganese sulfate.

24. The method of producing of claim 21, **characterized in that**, the fermentation culture step comprises: pH adjustment to 7.0±0.2 with 20%-30% (w/v) ammonia water; fermentation culture for 6-8 hours at an aeration rate of 0.8 vvm to 1.2 vvm and an initial agitation speed of 280 rpm to 320 rpm to an OD₆₀₀ value of 6.5-8.0, followed by stopping aeration; and continued fermentation culture for 50 to 70 hours at a modified agitation speed of 60 rpm to 100 rpm, followed by stopping fermentation; during fermentation process, the carbon source is supplemented when its concentration drops to a predetermined value;
preferably, during fermentation process, when the concentration of the carbon source drops to 30 g/L to 50 g/L, the carbon source is supplemented once so that the concentration of the carbon source becomes 80 g/L to 120 g/L;
preferably, during the fermentation process, when the concentration of the carbon source drops to 30 g/L to 50 g/L, a first supplementation of the carbon source is conducted to increase the concentration of the carbon source to 80 g/L to 120 g/L, and after that, when the concentration of the carbon source drops to 20 g/L to 40 g/L, a second supplementation of the carbon source is conducted to increase theconcentration of the carbon source to 60 g/L to 80 g/L.

25. The method of producing of claim 24, **characterized in that**, in the fermentation culture step, the carbon source is glucose.

26. The method of producing of any of claims 20 to 25, **characterized in that** the method of producing further comprises, prior to the fermentation culture step, obtaining activated seeds by seed culture and inoculating the activated seeds into fermentation culture medium for the fermentation culture.

27. The method of producing of claim 26, **characterized in that** the seed culture step comprises: inoculating the genetically engineered strain into seed culture medium and culturing it at a seed culture temperature of 42 °C to 55 °C for 12 h to 16 h, thus obtaining the activated seeds.

28. The method of producing of claim 26, **characterized in that**, in the seed culture step, the seed culture medium comprises following components: peptone, yeast powder and sodium chloride.
